# EUROPEAN PATENT SPECIFICATION

(11) **EP 2 547 675 B1**
(45) Date of publication and mention of the grant of the patent: **22.01.2014**
(21) Application number: 11710386.1
(22) Date of filing: 17.03.2011
(51) Int. Cl.: C08G 59/02

(54) **PROCESS FOR PREPARING DIVINYLARENE DIOXIDES**
VERFAHREN ZUR HERSTELLUNG VON DIVINYLARENDIOXIDEN
PROCÉDÉ DE PRÉPARATION DE DIOXYDES DE DIVINYLARÈNE

(30) Priority: 18.03.2010 US 315200 P
(43) Date of publication of application: 23.01.2013
(73) Proprietor: Dow Global Technologies LLC, Midland, MI 48674 (US)
(72) Inventor: GULYAS, Gyongyi, Lake Jackson TX 77566 (US); NULL, Marty, J., Lake Jackson TX 77566 (US)
(74) Representative: Raynor, John
(86) International application number: PCT/US2011/028794
(87) International publication number: WO 2011/116177

(56) References cited:
- EP-A1- 0 878 471
- EP-A2- 0 370 946
- WEIMING ZHU ET AL: "Oxidation of alkenes with aqueous potassium peroxymonosulfate and no organic solvent", THE JOURNAL OF ORGANIC CHEMISTRY, vol. 56, no. 25, 1 December 1991 (1991-12-01), pages 7022-7026, XP55000632, ISSN: 0022-3263, DOI: 10.1021/jo00025a014
- KACHASAKUL ET AL: "Extractive reaction for epoxidation of cyclohexane to cyclohexene oxide using dioxirane in ketone/Oxone system.", CHEMICAL ENGINEERING JOURNAL, vol. 92, 2003, pages 131-139, XP002661335, cited in the application

## Description

### BACKGROUND OF THE INVENTION

### Field of the Invention

The present invention is related to a process for preparing divinylarene dioxides, particularly divinylarene dioxides derived from divinylbenzene. More specifically, the present invention relates to a process for preparing a divinylarene dioxide by epoxidizing a divinylarene using a dioxirane.

### Description of Background and Related Art

Divinylarene dioxides, particularly divinylbenzene dioxide (DVBDO) and others which are derived from divinylbenzene (DVB) are a class of diepoxides which can be used as either a reactive diluent or as the main epoxy resin matrix in an epoxy thermoset formulation. DVBDO itself has a very low liquid viscosity (for example less than about 20 centipoise (0.02 Pas) making DVBDO especially useful in the preparation of low viscosity epoxy formulations. The epoxy formulations made from DVBDO are useful as intermediates in the production of various other products. For example, epoxy formulations made from DVBDO are suitable for use in the fields of coatings, composites, and molding compositions.

Previously known processes for the preparation of DVBDO include for example using hydrogen peroxide (H₂O₂) as the oxidant in the reaction process. However, none of these previously known prior art processes can produce DVBDO in high yields efficiently and economically (for example, greater than 30 percent (%) yield). For example, the process described in Inoue et al, Bull. Chem. Soc. Jap., 1991, 64, 3442, employs a molybdenum catalyst and sodium nitrate or sodium sulfate additives providing yields of DVBDO at less than 10 % because of product instability and catalyst deactivation.

Worzakowska M, J. Appl. Polym. Sci., 2007, 103, 462 discloses that DVBDO is prepared using a magnesium oxide catalyst in acetonitrile. Equimolar amounts of acetamide, an undesirable side-product, are co-produced in this known process. No isolated yields of DVBDO are mentioned; only the ratio of mono- and di-epoxide derivatives of DVB, which is 5, is mentioned.

There are also known processes for the preparation of DVBDO using other reactants which do not include H₂O₂. For example, a process for preparing DVBDO using peracetic acid is described in U.S. Patent No. 2,982,752. The epoxidation process disclosed in U.S. Patent No. 2,982,752 yields acetic acid side-product which can cause serious yield losses since divinyl arene oxides and dioxides show high acid sensitivity.

Hopff et al., Helvetica Chimica Acta, (1957), 40, 274; and German Patent No. DE 1079614 describes preparing DVBDO by reducing chloroacetylbenzenes with lithium aluminum hydride, and subsequently removing HCl from chlorohydrins in 32 % yield.

Sulfonium ylides for the preparation of epoxides from carbonyl compounds are described in Corey et al., J. Am. Chem. Soc., 1962, 84 (5), pp. 867-868. The reaction of sulfonium ylides and carbonyl compounds is successfully used for the synthesis of m-DVBDO from formaldehyde and m-(phenylenedimethylene)-dimethylsulfonium bromide in alkaline medium providing 70 % yield. However, the practical application of the above process is hampered by the long four-day production of the sulfonium bromide as described in U.S. Patent No. 3,455,967.

U.S. Patent No. 5,962,547 discloses the preparation of DVBDO using Oxone® (trademark of E. I. du Pont de Nemours and Company) in an acetone-water reaction mixture with no catalyst. The process described in U.S. Patent No. 5,962,547 produces DVBDO in quantitative yield. Oxone is a product that contains 1 mol KHSO₄ and 1 mol K₂SO₄ besides the active component of 2 mol KHSO₅. In U.S. Patent No. 5,962,547, the use of two mol excess of Oxone per mol of DVB is taught which means that four mol of KHSO₅ was used per mol of DVB, resulting in the generation of large amounts of sulfate co-products; eight mol of sulfates per mol DVB; four mol of KHSO₄ from the oxidant after it lost its active oxygen in addition to the four mol of additional salts that were originally present in Oxone.

The above processes known in the prior art all suffer from the disadvantage of generating residual products from the oxidizing agent that have to be separated from DVBDO; such as acids when peracids are the oxidants, or sulfides when sulphonium salts are used. Equivalent amount of acetamide is produced in the acetonitrile H₂O₂ system. Salts are co-products formed when chlorohydrins are treated with a base or when Oxone is used. In addition, the different known preparations usually provide low yields of DVBDO. The process disclosed by Corey et al. above describes a 70 % yield of DVBDO described; however, as aforementioned, that process is based on formaldehyde and a sulfonium salt, and such process requires an inconveniently long reaction time, for example as much as four days.

The disadvantages of above processes make such processes unsuitable for industrial scale preparation of divinylarene dioxides. Accordingly, it is desired to develop a process for the successful preparation of divinylarene dioxides using an oxidant and minimizing the co-production of undesirable side-products; and provide an economical and efficient process wherein the divinylarene dioxide is produced in high yields in an economical and efficient process.

### SUMMARY OF THE INVENTION

The present invention advantageously provides a process for successfully preparing divinylarene dioxides at high yields (e.g. greater than about 50 %), at high selectivities for epoxide formation, and at a relative lower cost than previous known processes without the problems of the prior art processes such as co-production of undesirable acidic side-products or large amounts of salts.

One embodiment of the present invention is directed to a process for preparing a divinylarene dioxide including reacting (a) at least one divinylarene; (b) at least one oxidant, wherein the oxidant is oxone and its molar ratio is less than 2.0 equivalents per mol of divinylarene; (c) at least one solvent; (d) at least one basic compound; and (e) optionally, at least one catalyst, under conditions to form a divinylarene dioxide product.

The present invention process for producing divinylarene dioxides uses a peroxomonosulfate triple salt oxidant, such as Oxone, suitable for obtaining high yields of a divinylarene dioxide. The present invention process is particularly suited for the preparation of divinylbenzene dioxide (DVBDO), a very low viscosity liquid epoxy resin, from divinylbenzene (DVB).

Advantageously, the present invention process is carried out under conditions such that the co-production of undesirable side-products is minimized. In addition, the process of the present invention advantageously produces divinylarene dioxides in high yields, for example, in yields of greater than about 50 %.

### BRIEF DESCRIPTION OF THE DRAWINGS

For the purpose of illustrating the present invention, the following drawings show a form of the present invention which is presently preferred. However, it should be understood that the present invention is not limited to the precise arrangements and apparatuses shown in the drawings. In the accompanying drawings, like reference numerals are used to denote like parts throughout the several drawings.
Figure 1 is a block flow diagram showing one embodiment of the overall process of the present invention using organic solvent extraction to separate the divinylarene dioxide product from the reaction effluent.
Figure 2 is a block flow diagram showing another embodiment of the overall process of the present invention using phase separation to separate the divinylarene dioxide product from the reaction effluent.

### DETAILED DESCRIPTION OF THE INVENTION

The present invention is directed to an economically favorable process for preparing a divinylarene dioxide. A divinylarene dioxide of the present invention is prepared by reacting a divinylarene with a peroxomonosulfate triple salt oxidant such as Oxone in the presence of a solvent, in the presence of a basic compound, and optionally in the presence of a catalyst, wherein the reagents and molar ratios of the reactants are selected to minimize the amounts of salt waste generated from the oxidant.

For example, in one embodiment of the present invention, a divinylarene dioxide such as divinylbenzene dioxide (DVBDO) may be prepared by dissolving a divinylarene such as divinylbenzene (DVB) in acetone, mixing the dissolved solution with a basic aqueous buffer, and using an aqueous solution of Oxone as the oxidizing agent. In this embodiment, acetone may also serve as a catalyst. Then the reaction may be carried out at a temperature of between about 0 °C to about 80 °C. After this epoxidation step is completed, the resulting product can be removed from the reaction mixture; and if desired, the resulting product may be purified by known means such as distillation.

In the economically favorable process of the present invention, the amount of side-products formed from the oxidizing agent is advantageously minimized. Oxone, useful in the process of the present invention as an oxidant, is also known as the "triple salt", since 1 mol Oxone is composed of 1 mol KHSO₄, 1 mol K₂SO₄ and 2 mol of KHSO₅, wherein the KHSO₅ is the active component. Consequently, an aqueous waste that results from carrying out the process contains KHSO₄; K₂SO₄ that is present in Oxone and KHSO₄ that forms after KHSO₅ loses its oxygen in the epoxidation reaction.

In order to minimize sulfate by-product formation in the process of the present invention, it is necessary to minimize the excess of the peroxomonosulfate triple salt oxidant such as Oxone that is used for epoxidation. However, Oxone for example, is usually used in excess compared to the double bond, since the epoxidation reaction is carried out at neutral pH, especially in case of acid sensitive epoxides. EP 0 878 471 A1 discloses a process for the oxidation of a divinylarene using Oxone wherein the molar ratio of oxidant: divinylarene is greater than 2. However, the_stability of the peroxomonosulfate triple salt oxidant such as Oxone dramatically decreases with increasing pH, as is well known in the prior art.

For the above stability issues, usually a 2-fold excess or more of the active component (KHSO5) of Oxone per double bond is used for epoxidations in prior art processes. For example, WO 02/18391 describes the epoxidation of a wide variety of olefins using different ketone catalysts and Oxone. In the general procedure of WO 02/18391, 1.8 mmol Oxone per mmol olefin is used which means 3.6 mmol of KHSO5 active oxidant is used per double bond. Similarly, Kachasakul et. al. Chem. Eng. Jour., 92, 2003, p 131 uses 1.15 mmol Oxone to epoxidize cyclohexane which means applying 2.3 mmol molar excess of oxidant over double bond. The epoxidation of several olefins are presented in Hashimoto et. al., Organic Proc. Res. And Dev., 6, 2002, p 405 using four fold excess of KHSO5 over double bond. Zhu et al. J. Org. Chem., 56, 1991, p 7022 describes the the oxidation of various alkenes using Oxone and shows that a higher concentration of KHSO5 per double bond results in increased product yields. EP 0370 946 A2 discloses the oxidation of allyl ethers using Oxone using a 4.2 mmol excess of oxidant per double bond. Therefore, the Oxone oxidant disclosed in the prior art can only be used in large excess (2-4 fold per double bond), when the oxidant is used for the preparation of acid sensitive epoxides; since the pH needs to be 7-8 where the stability of Oxone is diminished. Consequently, minimal excess of Oxone can only be used when the epoxidation reaction is faster than the decomposition of Oxone.

In its broadest scope, the present invention includes a process for preparing a divinylarene dioxide by reacting (a) at least one divinylarene; (b) at least one peroxomonosulfate triple salt oxidant, wherein the oxidant is less than 2.0 equivalents per mol of divinylarene; (c) a solvent, and (d) at least one basic compound. The process is carried out under conditions to form a divinylarene dioxide product. Optionally, the reaction mixture of the present invention process may include at least one catalyst; and optionally, other desirable additives.

The divinylarene and the other components above, are contacted with a peroxomonosulfate triple salt oxidant such as Oxone in a reactor, which may be batch or continuous; and the reactants are allowed to react to produce the corresponding divinylarene 30 dioxide. The co-produced salts, the other components left in the reactor, may be separated from the product to give a usable divinylarene dioxide product. The divinylarene dioxide product may optionally be purified, for example, by distillation, crystallization, or other purification methods known in the art. The solvent/catalyst can be recovered and recycled. To further improve process economics the sulfate salt by-product can be isolated, purified to the appropriate levels and used in different industrial and agricultural applications. Examples of industrial fields that use potassium sulfate include accelerator in the manufacture of wallboards; reagent component in paper pulping; flash suppressant for military applications; potassium supplement in food; component of an aqueous based drilling fluid and analytical reagent. The most important agricultural application is the use of potassium sulfate as a fertilizer; and to lesser extent in animal feed.

One of the examples of the present invention is directed to a process for preparation of DVBDO by epoxidation of DVB with dioxiranes, wherein the dioxiranes can be isolated or generated in-situ as described in R.W. Murray, J. Org. Chem., 50 (16) 2847, 1985, incorporated herein by reference. The in-situ generation of dioxiranes requires a ketone and a strong oxidizing agent. For example, the oxidizing agents can be peroxomonosulfate triple salt, such as Oxone. The ketone component acts as a catalyst and converts back to its original state after the oxidation reaction. For example, the ketone component can be chiral or achiral. Preferably, the simple achiral ketones, such as acetone, methyl-ethyl ketone, trifluoroacetone, acetophenone, tri or tetrafluoroacetopheneone, or mixtures thereof are used in the present invention.

In one embodiment, DVB and the optional ketone catalyst in an appropriate solvent and in the presence of an aqueous solution of the inorganic basic compound are treated with Oxone; and after the proper incubation time, DVBDO is obtained as the major reaction product with complete DVB conversion; with no or a very minor amount (less than about 10 %) of the monooxide of divinylbenzene (DVBMO) being formed. Then the reaction product can be extracted into an organic solvent, water washed, optionally filtered, then distilled to give a highly pure DVBDO product. The co-products are sulfates that can stay in the aqueous phase, or precipitate as solids, providing a very simple way of separation. The ketone component can be recycled and reused. It can be isolated from the reaction mixture by precipitation or extraction or distillation and the co-produced salts maybe purified and used in other industrial or agricultural applications.

As an illustration of one embodiment of the present invention, for example, a divinylarene dioxide such as divinylbenzene dioxide (DVBDO) is prepared by dissolving a divinylbenzene (DVB) in acetone. Acetone can also serve as the ketone catalyst and the solvent. The solution is mixed with an aqueous solution of the basic compound such as sodium hydrogencarbonate or solid sodium hydrogencarbonate can also be added. Then the oxidizing agent for example Oxone may be delivered to the reaction mixture; and then the reaction may be carried out at a temperature of between about 0 °C to about 80 °C. After the epoxidation is completed, the solvent, salts, and other materials may be removed from the product, and if desired, the product may be purified by known means such as distillation. The ketone component can be recycled and reused. It can be isolated from the reaction mixture by precipitation or extraction or distillation and the co-produced salts maybe purified and used in other industrial or agricultural applications.

In another embodiment, DVB in an appropriate solvent such as acetone and in the presence of an aqueous solution of the basic compound such as an inorganic salt or base are treated with the proper amount of oxidizing agent; and after the proper incubation time DVBDO is obtained as the major reaction product with complete DVB conversion; with minor amount less than about 40 % of the monoxide of divinylbenzene (DVBMO) being formed. Then the reaction product can be extracted into an organic solvent, water washed, optionally filtered, then the mono and di-epoxides are separated with appropriate methods such as distillation.

The source of divinylarene useful in the present invention may come from any known sources and particular to known processes for the preparation of divinylarenes. For example, divinylarenes can be prepared with salt or metal wastes from arenes and ethylene.

In one embodiment of the present invention, the divinylarene useful in the present invention may comprise any substituted or unsubstituted arene nucleus bearing two vinyl groups in any ring position. The arene may include for example benzene, substituted benzenes, or (substituted) ring-annulated benzenes, and mixtures thereof. In one embodiment, divinylbenzene may be *ortho*, *meta*, or *para* isomers or any mixture thereof. Additional substituents may consist of oxidation-resistant groups including for example saturated alkyl, aryl, halogen, nitro, isocyanate, or RO- (where R may be saturated alkyl or aryl), or mixtures thereof. Ring-annulated benzenes may include for example naphthalene, tetrahydronaphthalene, and the like, and mixtures thereof.

In another embodiment, the divinylarene may contain quantities of substituted arenes. The amount and structure of the substituted arenes depend on the process used in the preparation of the divinylarene. For example, DVB prepared by the dehydrogenation of diethylbenzene (DEB) may contain quantities of ethylvinylbenzene (EVB) and DEB.

The divinylarene used in the process, of the present invention may include for example divinylbenzene, divinylnaphthalene, divinylbiphenyl, divinyldiphenylether; and mixtures thereof.

The concentration of the divinylarene used in the present invention may range generally from about 0.1 weight percent (wt %) to about 70 wt %, preferably from about 1 wt % to about 60 wt %, and more preferably from about 5 wt % to about 50 wt %.

The oxidizing agent or oxidant useful in the present invention includes Oxone, Caroat® (trademark of Degussa), or other peroxomonosulfate triple salt that generates dioxirans in-situ with the ketone catalyst or isolated dioxirans can also be used.

The molar ratio of the oxidant used in the present invention is less than two equivalents per mol of divinylarene, preferably from about 1 equivalent to about 1.99 equivalents, and more preferably from about 1.1 equivalents to 1.5 equivalents.

The solvent useful in the process of the present invention may include for example any inert organic solvent that is inert to oxidant under the reaction conditions or it could be a ketone type solvent and besides organic solubilization it also can serve as the catalyst. For example, the solvent may include halogenated alkanes such as dichloromethane; aromatics such as toluene or xylene; hydrocarbons such as hexane or pentene; chlorinated solvents such as dichloromethane; polar organic solvents such as dimethyl formamide; nitriles such as acetonitrile, ketones such as acetone; ethers such as tetrahydrofuran or dimethoxy ethane; alcohols such as tert-amyl alcohol, tert-butanol, or methanol; fluorinated alcohols such as trifluoroethanol or hexafluroisopropanol; or mixtures thereof. Preferably, the solvent compound useful in the present invention may include for example, dioxane; dimethoxy ethane; acetonitrile; acetone; or mixture thereof.

The concentration of the solvent used in the present invention may range generally from about 1 wt % to about 99 wt %, preferably from about 25 wt % to about 75 wt %, and more preferably from about 35 wt % to about 60 wt %, based on the total weight of the composition.

The at least one basic compound useful in the present invention may include inorganic additives such as bases or salts that are capable of buffering the aqueous phase and providing an optimal pH for the reaction. For example, the basic compound can be sodium or potassium carbonate; hydrogen carbonate; sodium or potassium hydroxide; sodium or potassium phosphate; and mixtures thereof.

The concentration of the basic compound used in the present invention may range generally from 0.05 wt % to about 30 wt %, preferably from about 0.1 wt % to about 20 wt %, and more preferably from about 1 wt % to about 10 wt % based on the total weight of the composition.

The optional catalyst of the present invention may be selected from ketones or iminium salts. The ketone compound useful in the present invention can be chiral or achiral and may be for example, acetone, metyl-ethyl ketone, fluorinated ketones (trifluoroacetone, tri or tetrafluoro acetophenone), chiral ketones, and mixtures thereof.

Transition metal complexes could also be used as catalysts. Examples of useful transition metal complexes are Manganese Schiff base complexes such as Mn(III)-salen complexes.

The iminium catalyst of the present invention can also be chiral or achiral and can be selected from dihydroisoquinolinium, biphenylazepinium or binaphthalene-azepinium salts; and mixtures thereof. Typical examples of above structures are depicted as follows: 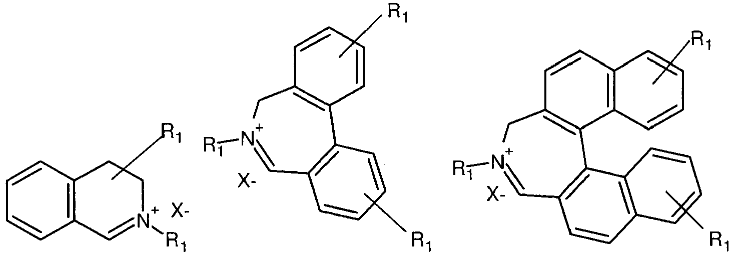 wherein R₁ can be hydrogen, alkyl, cycloalkyl, aryl, aralkyl groups; wherein the R₁ groups may also contain heteroatoms such as O or N; and X⁻ is halogen, tetrafluoroborate, tetraphenylborate, perchlorate and the like.

The iminium salts can also be generated in-situ from amines and aldehydes or ketones. An example of the amine component can be pyrrolidine or pyrrolidine substituted with electron withdrawing groups. The ketone component can be aromatic, aliphatic, or acyclic aldehydes and ketones and their substituted analogs, for example cyclohexanone, benzaldehyde, 2-chlorobenzaldehyde and the like.

The ratio of the catalyst in the present invention may range from 0 mol % to about 500 mol % (referenced to DVB), preferably from about 0.1 mol % to about 500 mol %, more preferably from about 1 mol % to about 200 mol %, most preferably from about 10 mol % to about 100 mol %.

Another optional component useful in the present invention may include for example a phase transfer agent. The phase transfer agent useful in the present invention may be for example, tetraakyl, tetraphenyl, or mixed alkyl-aryl ammonium or phosphonium salts; or crown ethers and mixtures thereof. Preferably, the phase transfer salts may include for example Bu₄NHSO₄, Bu₄NCl, 18-crown-6, and mixtures thereof.

The ratio of the optional phase transfer agent in the present invention may range from 0 mol % to about 25 mol % (referenced to DVB), preferably from about 0.1 mol % to about 25 mol %, more preferably from about 1 mol % to about 20 mol % and most preferably from about 2 mol % to about 10 mol %.

An example of another optional component useful in the present invention may include chelating agents to remove trace metal impurities to stabilize the oxidizing agent. The chelating agent useful in the present invention may be for example phosphates such as K₂HPO₄ or ethylenediamine tetraacetic acid (EDTA), diethylenetriamine pentaacetic acid (DTPA), and similar chelants with phosphonate groups such as ethylenediaminetetramethylene-tetraphosphonic acid (EDTMP) and the like. A generalized structure for the chelants is depicted as follows: 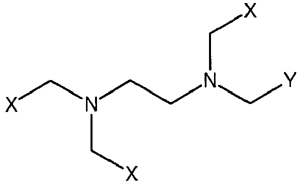 wherein X can be carboxylic acid or phosphonic acid groups or their alkali metal salts and Y can be X or -CH₂-N(CH₂-X)₂.

The concentration of the chelating agent used in the present invention may range generally from 0 wt % to about 20 wt %, preferably from about 0.01 wt % to about 10 wt %, and more preferably from about 0.05 wt % to about 5 wt %, based on the total weight of the composition.

Numerous additives can optionally be employed as part of the present invention including for example, a free radical polymerization inhibitor. For example, one or more free radical polymerization inhibitors may be added to any of the steps of the process of the present invention including for instance the reaction step, the recovery step and/or the purification step. The inhibitor may comprise a phenol; a hydroquinone; a quinone; an aromatic nitro compound, a nitrophenol, an amine; a nitroso compound; a nitroxide; or mixtures thereof.

Free radical polymerization inhibitors which may be employed in the present invention, include for example phenols such as 4-methoxy phenol, 4-tert-butylcatechol, or 2,6-di-tert-butyl-4-methylphenol; hydroquinones such as 1,4-dihyrdroxybenzene or 3,5-di-tert-butylbenzene-1,2-diol; quinones such as 1,4-benzoquinone or naphthalene-1,2-dione; aromatic nitro compounds such as 1,3-dinitrobenzene or 1,4-dinitrobenzene; nitrophenols such as 2-(sec-butyl)-4,6-dinitrophenol, 4-methyl-2-nitrophenol, or 4-methyl-2,6-dinitrophenol; amines such as phenothiazine, N¹-phenyl-N⁴-propylbenzene-1,4-diamine, N-(1,4-dimethylpentyl)-N'phenyl-p-phenylenediamine, N,N-diethylhydroxylamine; nitroso compounds such as N-nitrosophenylhydroxylamine ammonium salt; nitroxide compounds such as bis(1-oxyl-2,2,6,6-tetramethylpiperidin-4-yl) sebacate, 1-oxy-2,2,6,6-tetramethylpiperidine, 1-oxyl-2,2,6,6-tetramethylpiperidin-4-ol, 1-oxyl-2,2,6,6-tetramethyl-4-n-butoxypiperidine; or mixtures thereof.

The concentration of the inhibitors used in the present invention can be for example from about 0.01 wt % up to 5 wt % based on the divinylarene added. Preferably it should range from about 0.1 wt % up to 2 wt % based on the divinylarene added.

An assortment of optional additives may be added to the composition of the present invention including for example, other resins, stabilizers, fillers, plasticizers, catalyst de-activators, and the like; and mixtures thereof.

The concentration of the optional additives used in the present invention may range generally from 0 wt % to about 99.9 wt %, preferably from about 0.1 wt % to about 99.9 wt %, more preferably from about 1 wt % to about 99 wt %, and most preferably from about 2 wt % to about 98 wt %.

The preparation of divinylarene dioxides with the minimization of the co-production of undesirable side-products may be achieved by (i) adding to a reactor the following reactants: a divinylarene; a solvent; a basic compound or its aqueous solution; and optionally, a catalyst; (ii) contacting the reactants with a peroxomonosulfate triple salt oxidant, such as Oxone; and (iii) allowing the reactant components to react under reaction conditions to produce the corresponding divinylarene dioxide. In the process of the present invention, the basic compound may be added at the beginning of the reaction; simultaneously or intermittently with the oxidant.

The reaction conditions to manufacture the divinylarene dioxides, such as DVBDO, include carrying out the reaction of the reactants under a temperature, generally in the range of from about 0 °C to about 80 °C, preferably from about 5 °C to about 60 °C; more preferably from about 15 °C to about 50 °C; and most preferably from about 15 °C to about 30 °C. The pressure of the reaction may be generally from about 10.1 k Pa to about 1013 k Pa. The pH of the reaction may be controlled to a pH of from about 5 to about 12; preferably from about 6 to about 10 and more preferably from about 7 to about 8.

The reaction process of the present invention may be a batch or a continuous process. The reactor used in the process may be any reactor and ancillary equipment well known to those skilled in the art.

In addition, after the above reaction, the process of the present invention may include further processing steps such as a method of separating any co-products formed during the reaction from product. The separation method may include any separation process and equipment well known to those skilled in the art.

During the reaction for the preparation of divinylarene dioxides, an equivalent amount of potassium sulfate co-product that forms can be removed by (i) filtration with the separation of an organic phase and an aqueous phase; or by (ii) extraction of the divinylarene dioxide product with a non-miscible organic solvent followed by the appropriate water washes of the organic phase. Other undesirable oxidized by-products and derivatives, such as for example carbonyl compounds and hydrolyzed epoxy products, are not formed in any appreciable quantities using the process of the present invention.

After the reaction of the present invention, the undesirable co-products; and any remaining, catalyst, and solvent, may be removed to recover a usable divinylarene dioxide product. Then the product may optionally be purified by well-known means in the art such as by distillation, crystallization, precipitation, extraction and the like.

The solvent and catalyst components of the reaction mixture are separated from the divinylarene dioxide product by well-known means in the art such as by distillation, extraction and the like and recycled.

The salt by-product is separated from the reaction mixture by well-known means in the art such as by filtration. Residual organic content maybe removed by organic solvent washes, calcination, recrystallization, partial recrystallization or adsorption on activated carbon and the like.

In one embodiment of the process of the present invention, the process for preparing a divinylarene dioxide generally may comprise the steps of:
(A) contacting at least one divinylarene with Oxone in a pH controlled environment; optionally with other additives such as at least one solvent and at least one catalyst; to produce a divinylarene dioxide product in a reaction mixture;
(B) separating the divinylarene dioxide product formed in step (A) from the reaction mixture of step (A);
(C) optionally, recovering and/or recycling the solvent and catalyst from the reaction mixture of step (A); and
(D) optionally, recovering and purifying the sulfate by-product from the reaction mixture of step (A).

In other embodiments of the process of the present invention, the process for preparing a divinylarene dioxide may include one or more the following optional steps: (i) *in-situ* generation of dioxirane from a ketone and an oxidizing agent, such as Oxone in a pH controlled reaction in the presence of the divinyl arene. The pH can be controlled by introducing a buffer or a base into a reactor and then delivering the oxidizing agent to the reactor or by the simultaneously delivering the oxidizing agent and buffer or base. The basic compound can be added as a solid or as an aqueous solution; (ii) reacting an isolated dioxirane, prepared from a ketone and Oxone with a divinylarene such as DVB; (iii) separating sulfate co-product from a divinylarene dioxide product such as DVBDO product, by filtration and/or extraction of DVBDO with an organic solvent; (iv) removing a solvent and ketone from a divinylarene dioxide product such as DVBDO product by evaporation or distillation; (v) distilling a divinylarene dioxide product such as DVBDO product to give a high purity DVBDO product; (vi) recycling a ketone and solvent component by separating the ketone from a divinylarene dioxide product/solvent by precipitation, distillation or extraction; (vii) regenerating Oxone; or (viii) isolating and purifying the sulfate by-product by organic solvent washes; calcination; recrystallization or adsorption of the organic contaminants onto activated carbon.

As an illustration of another embodiment of the process of the present invention, the process for preparing a divinylarene dioxide may include one or more the following optional steps: (i) generating, *in-situ,* dioxirane from a catalyst, which can be a ketone, and an oxidizing agent, which is Oxone; in a pH controlled reaction in the presence of a divinylarene. The pH can be controlled by introducing a buffer or a basic compound into the reactor and then delivering the oxidizing agent; or by the simultaneous delivery of the oxidizing agent and buffer or base; (ii) using appropriate oxidizing agent and divinylarene molar ratios that results in the formation of a divinylarene dioxide as the main product accompanied by a divinylarene monooxide as the minor component; (iii) reacting an isolated dioxirane generated from a ketone and Oxone with a divinylarene such as DVB in a pH controlled environment; (iv) separating sulfate co-products from a divinylarene oxidation products such as DVBDO and DVBMO products, by filtration and/or extraction with an organic solvent; (v) removing a solvent and ketone from a divinylarene oxidation product such as DVBDO and DVBMO product; (vi) distilling a divinylarene mono and dioxide product mixture such as DVBDO and DVBMO products to give a high purity major DVBDO and minor DVBMO product; (vii) recycling a ketone and/or a solvent component by separating the ketone from a divinylarene dioxide product/solvent by precipitation, distillation or extraction; (viii) regenerating Caro's acid; (ix) isolating the sulfate by-product by filtration; (x) purifying the sulfate by-product by organic solvent washes or calcination or crystallization or adsorption of the impurities onto activated carbon.

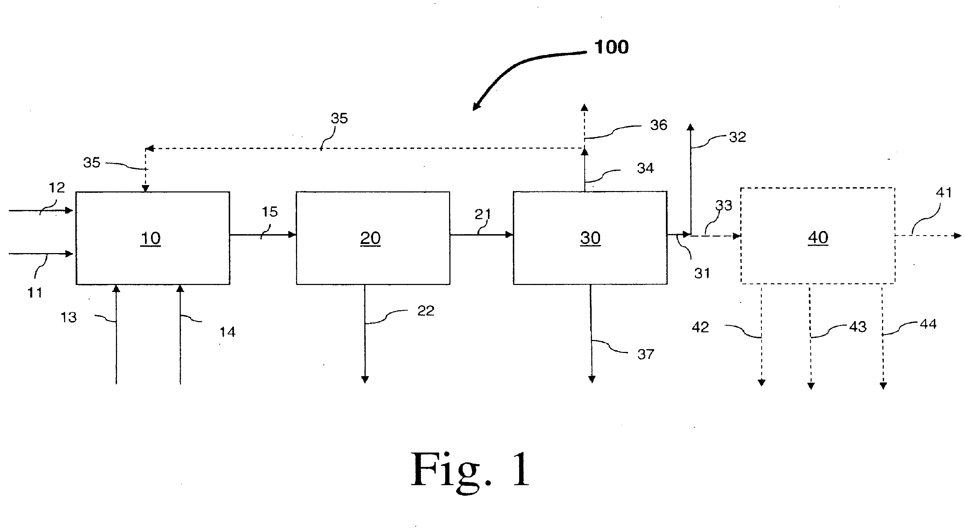

With reference to Figure 1, there is shown one embodiment of the process of the present invention generally indicated by numeral 100. The process of Figure 1 includes a reactor 10, a separation/filtration apparatus 20, an evaporation apparatus 30 and a purification/distillation apparatus 40. In Figure 1, there is shown a feed stream of divinylarene 11, a feed stream of ketone solvent and/or catalyst 12, a feed stream of a basic compound 13, and a feed stream of oxidant 14; all of the streams 11-14 which are fed into the reaction apparatus, reactor 10, for carrying out the epoxidation reaction step of the present invention. A recycle stream 34, 35 may also be introduced into reactor 10 (described below).

A product stream 15 exits from reactor 10 and may be introduced as a feed stream 15 to the separation/filtration apparatus 20, wherein solid and liquid components are separated. A liquid component stream 21 from apparatus 20 contains the divinylarene dioxide product, and stream 21 is sent for further processing to apparatus 30. A solid reaction component stream 22 (which may include a salt byproduct) is separated from the product liquid stream 21 and stream 22 exits apparatus 20.

The divinylarene dioxide product stream, stream 21 can be sent to the solvent removal/evaporator apparatus 30, for separation of the product from solvent/catalyst. The product stream from apparatus 30, stream 31, may be used without further purification as stream 31, 32; or optionally, as shown in dotted lines, the product stream 31 may be introduced to a purification/distillation apparatus 40 as feed stream 31, 33. An organic stream containing the solvent/catalyst from apparatus 30, stream 34, may be removed from apparatus 30 and optionally recycled to reactor 10 as stream 34, 35. Optionally, stream 34 may be purged as stream 34, 36 or sent to an organic waste recovery unit not shown. An aqueous waste stream from reactor 30, stream 37 can be sent to a waste water treatment unit (not shown)

As aforementioned, the divinylarene dioxide product stream from apparatus 30, stream 31, 33, may optionally be introduced to a purification/distillation apparatus 40, to form a purified product stream, stream 41. Also if divinylarene monooxide accompanies the divinylarene dioxide product, the divinylarene monoxide can be separated as a byproduct stream 42 in the purification/distillation apparatus 40. The divinylarene monooxide byproduct leaves apparatus 40 as stream 42 and distillation bottoms as stream 43. Possible contaminants with lower boiling point than DVBDO, for example alkylvinylbenzene oxides or low levels of oxidation by-products can also be separated and removed from apparatus 40 as stream 44.

Any of the recycle streams of Figure 1 may require a periodic or continuous purge to limit the buildup of impurities.

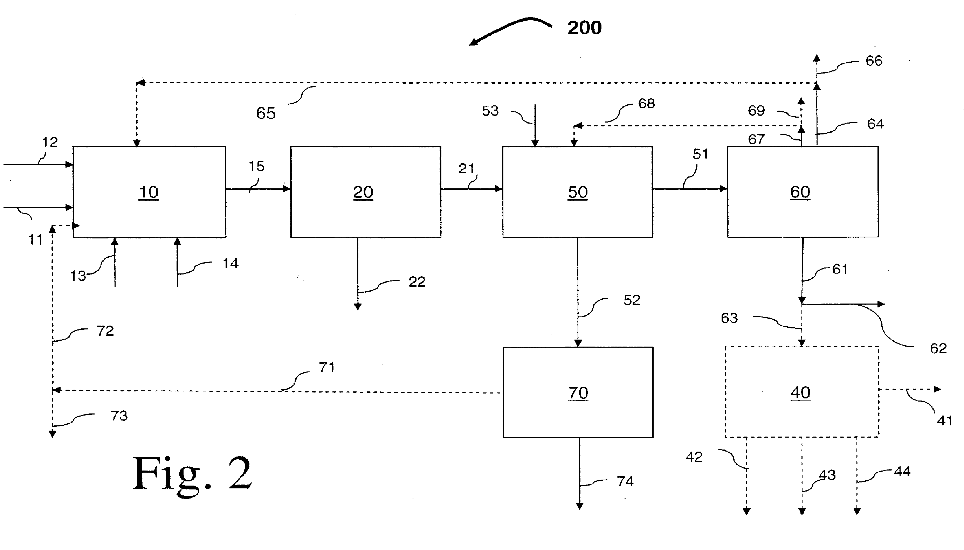

With reference to Figure 2, there is shown another embodiment of the process of the present invention generally indicated by numeral 200. The process of Figure 2 includes a reactor 10 similar to that shown in Figure 1, a separation/filtration apparatus 20 similar to that shown in Figure 1, an extraction apparatus 50, a separation apparatus 60, a purification/distillation apparatus 40 similar to that shown in Figure 1, and a separation apparatus 70. In Figure 2, there is shown a feed stream of divinylarene 11, a feed stream of ketone catalyst and/or solvent 12, a feed stream of a basic compound 13, and a feed stream of oxidant 14. All of which are fed into the reaction apparatus, reactor 10, for carrying out the epoxidation reaction step of the present invention. A recycle stream 73 also is introduced into reactor 10 (described below).

The product stream 15 from reactor 10 may be introduced as feed stream 15 to the separation/filtration apparatus 20, wherein in the divinylarene dioxide product stream 21 also containing catalyst /solvent, is separated from the salt by-product stream 22. In the embodiment shown in Figure 2, the divinylarene dioxide product stream, stream 21, is sent to the extraction apparatus 50.

Feed stream 21 is introduced into extraction apparatus 50, for separation from the aqueous and aqueous soluble components by extraction with an organic extraction solvent, stream 53 and a recycled extraction solvent, stream 67, 68. The extracted product in the extraction solvent is separated from the aqueous phase and may be introduced to the separation apparatus 60 as stream 51. An aqueous organic waste stream from apparatus 50, stream 52, may also be removed and introduced to the separator apparatus 70.

Stream 51, may be introduced to separation apparatus 60, to separate from the extraction solvent. The purified divinylarene product stream, stream 61 leaving apparatus 60, can be used as is without further processing, as stream 61, 62; or optionally, as shown in dotted lines, the stream 61 may be used for applications requiring high purity product and thus may be purified further and fed as stream 61, 63 in a purification/distillation apparatus, apparatus 40. The extraction solvent, stream 67, 68 can be recycled into apparatus 50. Optionally, stream 67 may be purged as stream 67, 69 or sent to an organic waste recovery unit not shown. In another embodiment, the extraction solvent might dissolve some of the reaction solvent/catalyst which also can be separated from the extraction solvent in apparatus 60 and recycled to reactor 10 as stream 64, 65. Optionally, stream 64 may be purged as stream 64, 66 or sent to an organic waste recovery unit not shown.

Product stream 61, 63 can be further purified in a purification/distillation apparatus, such as apparatus 40. Also if divinylarene monooxide accompanies the divinylarene dioxide product, the monooxide can also be separated in purification/distillation apparatus 40. High purity divinylarene product leaves apparatus 40 as stream 41, divinylarene monooxide byproduct as stream 42 and distillation bottoms as stream 43. Possible contaminants with lower boiling point than DVBDO, for example alkylvinylbenzene oxides or low levels of oxidation by-products can also be separated and removed from apparatus 40 as stream 44.

Stream 52, the aqueous phase from apparatus 50, might contain catalyst/solvent. Strem 52 can be fed into the separation apparatus 70, to separate the water from the solvent/catalyst component. From apparatus 70, the solvent/catalyst component can be recycled as stream 71, 72 back to the epoxidation reactor 10. Optionally, stream 71 may be purged as stream 71, 73 or sent to an organic waste recovery unit not shown. An aqueous waste stream from reactor 70, stream 74, can be sent to a waste water treatment unit (not shown)

Any of the recycle streams of Figure 2 may require a periodic or continuous purge to limit the buildup of impurities.

One advantage of the present invention process is that high yields of divinylarene dioxides may be produced by the process of the present invention. With high yields of divinylarene dioxides produced, the process of the present invention advantageously requires no recycle of divinylarene or divinylarene monooxide.

The "high yield" of the divinylarene dioxides produced by the process of the present invention, is generally greater than about 50 %; and preferably, ranges from about 70 % to about 100 %; more preferably, from about 80 % to about 100 %; and most preferably, from about 90 % to about 100 % based on divinylarene starting material.

Another advantage of the present invention is the use of a minimal molar excess of peroxomonosulfate triple salt oxidant compared to divinylarene which reduces sulfate by-product generation by at least about 45 %.

The divinylarene dioxides prepared by the process of the present invention, particularly those derived from divinylbenzene such as for example divinylbenzene dioxide (DVBDO), are class of diepoxides which have a relatively low liquid viscosity but a higher rigidity than conventional epoxy resins.

The divinylarene dioxide prepared by the process of the present invention may comprise, for example, any substituted or unsubstituted arene nucleus bearing two vinyl groups in any ring position. The arene portion of the divinylarene dioxide may comprise benzene, substituted benzenes, ring-annulated benzenes, substituted ring-annulated benzenes, or mixtures thereof. The divinylarene portion of the divinylarene dioxide may be *ortho*, *meta*, or *para* isomers or any mixture thereof. Additional substituents may consist of H₂O₂-resistant groups including saturated alkyl, aryl, halogen, nitro, isocyanate, or RO- (where R may be a saturated alkyl or aryl). Ring-annulated benzenes may comprise for example naphthlalene, tetrahydronaphthalene, and the like. Homologously bonded (substituted) benzenes may comprise for example biphenyl, diphenylether, and the like.

The divinylarene oxide product prepared by the process of the present invention may be illustrated generally by general chemical Structures I -IV as follows:

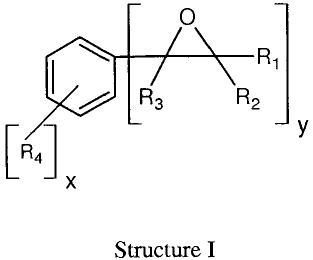

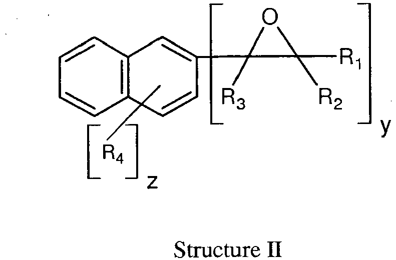

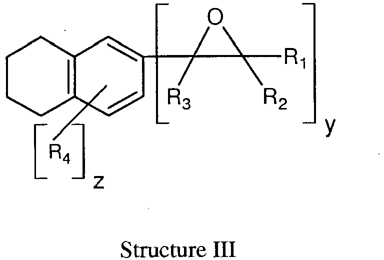

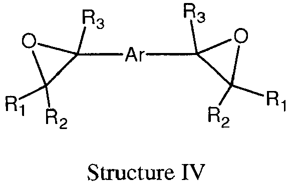

In the above Structures I, II, III and IV of the divinylarene dioxide product of the present invention, each R₁, R₁, R₃ and R₄ individually may be hydrogen, an alkyl, cycloalkyl, an aryl or an aralkyl group; or a oxidant-resistant group including for example a halogen, a nitro, an isocyanate, or an RO group, wherein R may be an alkyl, aryl or aralkyl; x may be an integer of 0 to 4; y may be an integer greater than or equal to 2; x+y may be an integer less than or equal to 6; z may be an integer of 0 to 6; and z+y may be an integer less than or equal to 8; and Ar is an arene fragment including for example, 1,3-phenylene group.

The divinylarene dioxide product produced by the process of the present invention may include for example alkyl-vinyl-arene monoxides depending on the presence of alkylvinylarene in the starting material.

In one embodiment of the present invention, the divinylarene dioxide produced by the process of the present invention may include for example divinylbenzene dioxide, divinylnaphthalene dioxide, divinylbiphenyl dioxide, divinyldiphenylether dioxide, and mixtures thereof.

In a preferred embodiment of the present invention, the divinylarene dioxide used in the epoxy resin formulation may be for example divinylbenzene dioxide (DVBDO). Most preferably, the divinylarene dioxide component that is useful in the present invention includes, for example, a divinylbenzene dioxide as illustrated by the following chemical formula of Structure V:

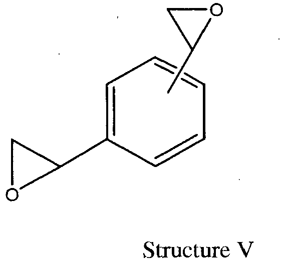

The chemical formula of the above DVBDO compound may be as follows: C₁₀H₁₀O₂; the molecular weight of the DVBDO is about 162.2; and the elemental analysis of the DVBDO is about: C, 74.06; H, 6.21; and O, 19.73 with an epoxide equivalent weight of about 81 g/epoxide equivalent.

Divinylarene dioxides, particularly those derived from divinylbenzene such as for example divinylbenzene dioxide (DVBDO), are class of diepoxides which have a relatively low liquid viscosity but a higher rigidity and crosslink density than conventional epoxy resins.

Structure VI below illustrates an embodiment of a preferred chemical structure of a divinylbenzene dioxide (DVBDO) useful in the present invention:

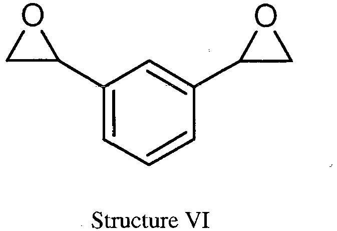

Structure VII below illustrates another embodiment of a preferred chemical structure of the DVBDO useful in the present invention:

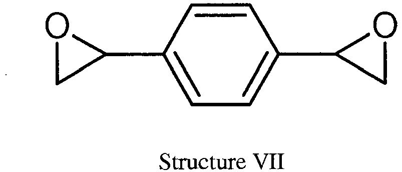

When DVBDO is prepared by the process of the present invention, it is possible to obtain one of three possible isomers: *ortho*, *meta*, and *para.* Accordingly, the present invention includes a DVBDO illustrated by any one of the above Structures individually or as a mixture thereof. Structures VI and VII above show the *meta* (1,3-DVBDO) isomer and the *para* (1,4-DVBDO) isomer of DVBDO, respectively. The *ortho* isomer is rare; and usually DVBDO is mostly produced generally in a range of from about 9:1 to about 1:9 ratio of *meta* (Structure VI) to *para* (Structure VII) isomers. The present invention preferably includes as one embodiment a range of from about 6:1 to about 1:6 ratio of Structure VI to Structure VII, and in other embodiments the ratio of Structure VI to Structure VII may be from about 4:1 to about 1:4 or from about 2:1 to about 1:2.

In one embodiment, the process of the present invention is particularly suited for the preparation of divinylbenzene dioxide, a low viscosity liquid epoxy resin. The viscosity of the divinylarene dioxides produced by the process of the present invention ranges generally from about 10 mPa-s to about 100 mPa-s; preferably, from about 10 mPa-s to about 50 mPa-s; and more preferably, from about 10 mPa-s to about 25 mPa-s at 25 °C.

The utility of the divinylarene dioxides of the present invention requires their thermal stability to allow their formulation or processing at moderate temperatures (for example, at from about 100 °C to about 200 °C) for up to several hours (for example, for at least 2 hours) without oligomerization or homopolymerization. Oligomerization or homopolymerization during formulation or processing is evident by a substantial increase in viscosity or gelling (crosslinking). The divinylarene dioxides of the present invention have sufficient thermal stability such that they do not experience a substantial increase in viscosity or gelling during formulation or processing at moderate temperatures.

The divinylarene dioxide products of the present invention are useful for the preparation of epoxy resin compositions or formulations which, in turn, are useful for preparing thermosets or cured products in the form of coatings, films, adhesives, laminates, composites, electronics, and the like.

As an illustration of the present invention, in general, resin compositions based on the divinylarene dioxide products of the present invention may be useful for casting, potting, encapsulation, molding, and tooling. The present invention is particularly suitable for all types of electrical casting, potting, and encapsulation applications; for molding and plastic tooling; and for the fabrication of vinyl ester resin based composites parts, particularly for producing large vinyl ester resin-based parts produced by casting, potting and encapsulation. The resulting composite material may be useful in some applications, such as electrical casting applications or electronic encapsulations, castings, moldings, potting, encapsulations, injection, resin transfer moldings, composites, coatings and the like.

### EXAMPLES

The following examples and comparative examples further illustrate the present invention in detail but are not to be construed to limit the scope thereof. The product mixtures prepared in the Examples which follow were analyzed by standard gas chromatography (GC) analytical equipment and methods.

Various terms and designations used in the following examples are explained herein as follows: "DVB" stands for divinylbenzene; "DVBDO" stands for divinylbenzene dioxide; "DVBMO" stands for divinylbenzene monoxide; and "EVB" stands for ethylvinylbenzene, DME stands for dimethoxyethane.

For each of the following preparations in the Examples, 80 % DVB was used containing 20 % EVB but the yields and final compositions were referred to DVB. All chemicals were purchased from Sigma-Aldrich and used without further purifications.

### Example 1

DVB (781 mg, 6 mmol), dimethoxyethane (47 mL) and acetone (23 mL) were transferred to a flask equipped with a mechanical stirrer, thermocouple and pH meter. Sodium hydrogen carbonate (3024 mg, 36 mmol) was dissolved in water (38 mL) and was also transferred into the flask. Oxone (5.52 g, 9 mmol) was dissolved in water (38 mL). Oxone solution was delivered into the reaction mixture in the course of 15 minutes maintaining the temperature at 25 °C. The pH of the reaction was continuously monitored and it was between 7 and 8. The reaction mixture was further incubated at this temperature and was analyzed by GC. After two hours from the start of the oxidant addition, DVB was completely converted to products. There was no DVBMO present and the reaction mixture contained 99 % DVBDO.

### Example 2

DVB (11.2 g, 86 mmol), acetone (750 mL) and a solution of sodium hydrogen carbonate (43.3g, 520 mmol) in water (380 mL) were transferred to a flask equipped with a mechanical stirrer, thermocouple and pH meter. Oxone® (58 g, 95 mmol) was dissolved in water (320 mL) and delivered into the reaction mixture in the course of 35 minutes maintaining the temperature at 25 °C and stirring at 350 rpm. The pH of the solution was continuously monitored and was at 7-8. The reaction mixture was further incubated for two hours. After 2 hours solids were filtered and washed with dichloromethane (250 mL). The filtrate was extracted with methylene chloride (250 mL). The organic layer of the filtrate was mixed with the dichloromethane wash and washed with sodium bicarbonate (250 mL, 8 % solution) then with water (250 mL). The organic layer was dried over sodium sulfate. The solvents were removed with rotary evaporation. Product yield was 95 %. The product was also analyzed by GC. DVB was completely converted to products and no DVBMO was present. The mixture was vacuum distilled to separate from EVBMO. Vacuum distillation resulted in a 10 % DVBDO loss; and 98 % pure DVBDO.

### Example 3

DVB (781 mg, 6 mmol), dimethoxyethane (47 mL) and acetonitrile (23 mL) were transferred to a flask equipped with a mechanical stirrer, thermocouple and pH meter. Sodium hydrogen carbonate (3024 mg, 36 mmol) was dissolved in water (38 mL) and was also transferred into the flask. Oxone (5.52 g, 9 mmol) was dissolved in water (38 mL). Oxone solution was delivered into the reaction mixture in the course of 15 minutes maintaining the temperature at 25 °C. The pH of the reaction was continuously monitored and it was between 7-8. The reaction mixture was further incubated at this temperature and was analyzed by GC after two hours from the start of the oxidant addition. 24 % DVBMO and 2 % DVBDO formation was obtained.

## Claims

1. A process for preparing a divinylarene dioxide comprising reacting (a) a divinylarene; (b) a peroxomonosulfate triple salt oxidant, wherein the oxidant is less than 2.0 equivalents per mol of divinylarene; (c) a solvent, and (d) a basic compound, under conditions to form a divinylarene dioxide product.

2. The process of claim 1, wherein the divinylarene is divinylbenzene;
wherein the divinylarene dioxide formed is divinylbenzene dioxide; wherein the oxidant is oxone; wherein the solvent comprises halogenated alkanes; aromatics; polar organic solvents; ethers; alcohols; fluorinated alcohols; ketones; or mixtures thereof; and wherein the basic compound comprises an inorganic base or an inorganic salt.

3. The process of claim 1, wherein the triple salt oxidant is mixed with a ketone catalyst which results in the formation of a dioxirane oxidant; wherein the dioxirane is isolated and then used in a subsequent process; or wherein the dioxirane is formed and used in situ as the oxidant in the presence of the divinylarene mixed with the catalyst during the addition of the triple salt.

4. The process of claim 1, wherein the solvent comprises dichloromethane; toluene; acetonitrile; dimethoxyethane, dioxane; methanol; trifluoroethanol; acetone; methyl-ethyl ketone or mixtures thereof.

5. The process of claim 1, wherein the basic compound comprises sodium hydrogen carbonate, sodium carbonate, sodium hydroxide, potassium hydroxide, potassium hydrogen carbonate, potassium carbonate, sodium phosphate, potassium phosphate, or mixtures thereof.

6. The process of claim 1, wherein the reaction is carried out at a temperature within the range of from about 0 °C to about 80 °C; and wherein the pH of the reaction is from about 5 to about 12.

7. The process of claim 1, including a catalyst; and wherein the catalyst comprises a ketone selected from a fluorinated ketone, an aliphatic ketone, an aromatic ketone, an aliphatic-aromatic ketone, a chiral ketone, or mixtures thereof; an iminium salt selected from dihydroisoquinolinium, biphenylazepinium or binaphthalene-azepinium salts, or mixtures thereof; or mixtures thereof.

8. The process of claim 7, wherein the ketone comprises acetone; methyl-ethyl ketone; acetophenone; trifluoroacetone; trifluoro acetophenone; 5 tetrafluoro acetophenone; or mixtures thereof.

9. The process of claim 7, wherein the iminium salt catalyst is generated in-situ from amines and aldehydes or ketones such as pyrrolidine or pyrrolidine substituted with electron withdrawing groups and cyclohexanone or 2-chlorobenzaldehyde.

10. The process of claim 7, wherein the catalyst is a ketone; and wherein the solvent is a ketone.

11. The process of claim 1, including a phase transfer agent; and wherein the phase transfer agent comprises an alkylammonium salt; an arylammonium salt; an alkylphosphonium salt; an arylphosphonium salt; a crown ether; or mixtures thereof

12. The process of claim 11, wherein the phase transfer agent comprises tetrabutylammonium hydrogen phosphate or 18-crown-6.

13. The process of claim 1, including adding one or more free radical polymerization inhibitors to the reaction mixture.

14. The process of claim 1, including a metal scavenging agent; and wherein the metal scavenging agent comprises ethylenediamine tetraacetic acid; diethylenetriamine pentaacetic acid; ethylenediaminetetramethylene-tetraphosphonic acid, sodium hydrogenphosphate, potassium hydrogenphosphate; sodium phosphate, potassium phosphate; or mixtures thereof.

15. A process for preparing a divinylarene dioxide comprising the steps of:
(A) contacting at least one divinylarene with a peroxomonosulfate triplesalt oxidant, wherein the oxidant is less than 2.0 equivalents per mol of divinylarene; a solvent; a basic compound; and optionally, with at least one catalyst; to produce a divinylarene dioxide product in a reaction mixture;
(B) separating the divinylarene dioxide product formed in step (A) from the reaction mixture of step (A);
(C) optionally, purifying the divinylarene dioxide product obtained in step (B);
(D) optionally, recovering and/or recycling the solvent 5 and catalyst from the reaction mixture of step (A); and
(E) optionally, recovering and purifying the by-product salt.

## Patentansprüche

1. Ein Verfahren zum Herstellen eines Divinylarendioxids, beinhaltend Zur-Reaktion-Bringen (a) eines Divinylarens; (b) eines Peroxomonosulfat-Tripelsalz-Oxidationsmittels, wobei das Oxidationsmittel weniger als 2,0 Äquivalente pro Mol Divinylaren beträgt; (c) eines Lösungsmittels und (d) einer basischen Verbindung bei Bedingungen, um ein Divinylarendioxidprodukt zu bilden.

2. Verfahren gemäß Anspruch 1, wobei das Divinylaren Divinylbenzol ist; wobei das gebildete Divinylarendioxid Divinylbenzoldioxid ist; wobei das Oxidationsmittel Oxone ist; wobei das Lösungsmittel halogenierte Alkane; Aromaten; polare organische Lösungsmittel; Ether; Alkohole; fluorierte Alkohole; Ketone oder Mischungen davon beinhaltet; und wobei die basische Verbindung eine anorganische Base oder ein anorganisches Salz beinhaltet.

3. Verfahren gemäß Anspruch 1, wobei das Tripelsalz-Oxidationsmittel mit einem Keton-Katalysator vermischt wird, was in der Bildung eines Dioxiran-Oxidationsmittels resultiert; wobei das Dioxiran isoliert wird und dann in einem nachfolgenden Verfahren verwendet wird; oder wobei das Dioxiran in situ gebildet und verwendet wird, als Oxidationsmittel in Gegenwart des Divinylarens, vermischt mit dem Katalysator während der Zugabe des Tripelsalzes.

4. Verfahren gemäß Anspruch 1, wobei das Lösungsmittel Dichlormethan; Toluol; Acetonitril; Dimethoxyethan, Dioxan; Methanol; Trifluorethanol; Aceton; Methylethylketon oder Mischungen davon beinhaltet.

5. Verfahren gemäß Anspruch 1, wobei die basische Verbindung Natriumhydrogencarbonat, Natriumcarbonat, Natriumhydroxid, Kaliumhydroxid, Kaliumhydrogencarbonat, Kaliumcarbonat, Natriumphosphat, Kaliumphosphat oder Mischungen davon beinhaltet.

6. Verfahren gemäß Anspruch 1, wobei die Reaktion bei einer Temperatur im Bereich von etwa 0 °C bis etwa 80 °C durchgeführt wird; und wobei der pH-Wert der Reaktion von etwa 5 bis etwa 12 beträgt.

7. Verfahren gemäß Anspruch 1, einschließlich eines Katalysators; und wobei der Katalysator ein Keton, das ausgewählt ist aus einem fluorierten Keton, einem aliphatischen Keton, einem aromatischen Keton, einem aliphatisch-aromatischen Keton, einem chiralen Keton oder Mischungen davon; ein Iminiumsalz, das ausgewählt ist aus Dihydroisochinolinium-, Biphenylazepinium- oder Binaphthylazepiniumsalz oder Mischungen davon; oder Mischungen davon beinhaltet.

8. Verfahren gemäß Anspruch 7, wobei das Keton Aceton; Methylethylketon; Acetophenon; Trifluoraceton; Trifluoracetophenon; 5-Tetrafluoracetophenon oder Mischungen davon beinhaltet.

9. Verfahren gemäß Anspruch 7, wobei der Iminiumsalz-Katalysator in situ aus Aminen und Aldehyden oder Ketonen, wie Pyrrolidin oder Pyrrolidin, substituiert mit elektronenziehenden Gruppen und Cyclohexanon oder 2-Chlorbenzaldehyd, erzeugt wird.

10. Verfahren gemäß Anspruch 7, wobei der Katalysator ein Keton ist; und wobei das Lösungsmittel ein Keton ist.

11. Verfahren gemäß Anspruch 1, einschließlich eines Phasentransfermittels; und wobei das Phasentransfermittel ein Alkylammoniumsalz; ein Arylammoniumsalz; ein Alkylphosphoniumsalz; ein Arylphosphoniumsalz; einen Kronenether oder Mischungen davon beinhaltet.

12. Verfahren gemäß Anspruch 11, wobei das Phasentransfermittel Tetrabutylammonium-Hydrogenphosphat oder 18-Krone-6 beinhaltet.

13. Verfahren gemäß Anspruch 1, einschließlich des Hinzufügens eines oder mehrerer Hemmstoffe der Polymerisation durch freie Radikale zu der Reaktionsmischung.

14. Verfahren gemäß Anspruch 1, einschließlich eines Metalldesoxidationsmittels; und wobei das Metalldesoxidationsmittel Ethylendiamintetraessigsäure; Diethylentriaminpentaessigsäure; Ethylendiamintetramethylentetraphosphonsäure, Natriumhydrogenphosphat, Kaliumhydrogenphosphat; Natriumphosphat, Kaliumphosphat oder Mischungen davon beinhaltet.

15. Ein Verfahren zum Herstellen eines Divinylarendioxids, beinhaltend die folgenden Schritte:
(A) In-Kontakt-Bringen mindestens eines Divinylarens mit einem Peroxomonosulfat-Tripelsalz-Oxidationsmittel, wobei das Oxidationsmittel weniger als 2,0 Äquivalente pro Mol Divinylaren beträgt; einem Lösungsmittel; einer basischen Verbindung; und optional mit mindestens einem Katalysator, um ein Divinylarendioxidprodukt in einer Reaktionsmischung zu bilden;
(B) Trennen des Divinylarendioxidprodukts, das in Schritt (A) gebildet wurde, von der Reaktionsmischung aus Schritt (A);
(C) optional, Reinigen des Divinylarendioxidprodukts, das in Schritt (B) erhalten wurde;
(D) optional, Rückgewinnen und/oder Rezyklieren des Lösungsmittels 5 und des Katalysators aus der Reaktionsmischung aus Schritt (A); und
(E) optional, Rückgewinnen und Reinigen des Abproduktsalzes.

## Revendications

1. Un procédé de préparation d'un dioxyde de divinylarène comprenant la réaction entre (a) un divinylarène ; (b) un oxydant de type sel triple de peroxomonosulfate, l'oxydant étant inférieur à 2,0 équivalents par mole de divinylarène ; (c) un solvant, et (d) un composé basique, dans des conditions pour former un produit de dioxyde de divinylarène.

2. Le procédé de la revendication 1, dans lequel le divinylarène est du divinylbenzène ; dans lequel le dioxyde de divinylarène formé est du dioxyde de divinylbenzène ; dans lequel l'oxydant est de l'oxone ; dans lequel le solvant comprend des alcanes halogénés ; des composés aromatiques ; des solvants organiques polaires ; des éthers ; des alcools ; des alcools fluorés ; des cétones ; ou des mélanges de ceux-ci ; et dans lequel le composé basique comprend une base inorganique ou un sel inorganique.

3. Le procédé de la revendication 1, dans lequel l'oxydant de type sel triple est mélangé avec un catalyseur de type cétone, résultant en la formation d'un oxydant de type dioxirane ; dans lequel le dioxirane est isolé et utilisé ensuite dans un procédé ultérieur ; ou dans lequel le dioxirane est formé et utilisé in situ en tant qu'oxydant en présence du divinylarène mélangé avec le catalyseur durant l'ajout du sel triple.

4. Le procédé de la revendication 1, dans lequel le solvant comprend du dichlorométhane ; du toluène ; de l'acétonitrile ; du diméthoxyéthane, du dioxane ; du méthanol ; du trifluoroéthanol ; de l'acétone ; de la méthyléthylcétone ou des mélanges de ceux-ci.

5. Le procédé de la revendication 1, dans lequel le composé basique comprend de l'hydrogénocarbonate de sodium, du carbonate de sodium, de l'hydroxyde de sodium, de l'hydroxyde de potassium, de l'hydrogénocarbonate de potassium, du carbonate de potassium, du phosphate de sodium, du phosphate de potassium, ou des mélanges de ceux-ci.

6. Le procédé de la revendication 1, dans lequel la réaction est effectuée à une température comprise dans la gamme allant d'environ 0 °C à environ 80 °C ; et dans lequel le pH de la réaction va d'environ 5 à environ 12.

7. Le procédé de la revendication 1, comportant un catalyseur ; et dans lequel le catalyseur comprend une cétone sélectionnée parmi une cétone fluorée, une cétone aliphatique, une cétone aromatique, une cétone aliphatique-aromatique, une cétone chirale, ou des mélanges de celles-ci ; un sel d'iminium sélectionné parmi des sels de dihydroisoquinolinium, biphénylazépinium ou binaphthalène-azépinium, ou des mélanges de ceux-ci ; ou des mélanges de ceux-ci.

8. Le procédé de la revendication 7, dans lequel la cétone comprend de l'acétone ; de la méthyléthylcétone ; de l'acétophénone ; de la trifluoroacétone ; de la trifluoroacétophénone ; de la 5-tétrafluoroacétophénone ; ou des mélanges de celles-ci.

9. Le procédé de la revendication 7, dans lequel le catalyseur de type sel d'iminium est généré in situ à partir d'amines et d'aldéhydes ou de cétones comme la pyrrolidine ou la pyrrolidine substituée avec des groupes accepteurs d'électrons et de la cyclohexanone ou du 2-chlorobenzaldéhyde.

10. Le procédé de la revendication 7, dans lequel le catalyseur est une cétone, et dans lequel le solvant est une cétone.

11. Le procédé de la revendication 1, comportant un agent de transfert de phase ; et dans lequel l'agent de transfert de phase comprend un sel d'alkylammonium ; un sel d'arylammonium ; un sel d'alkylphosphonium ; un sel d'arylphosphonium ; un éther couronne ; ou des mélanges de ceux-ci.

12. Le procédé de la revendication 11, dans lequel l'agent de transfert de phase comprend de l'hydrogénophosphate de tétrabutylammonium ou du 18-couronne-6.

13. Le procédé de la revendication 1, comportant l'ajout d'un ou de plusieurs inhibiteurs de polymérisation à radicaux libres au mélange réactionnel.

14. Le procédé de la revendication 1, comportant un agent de piégeage de métal ; et dans lequel l'agent de piégeage de métal comprend de l'acide tétraacétique d'éthylènediamine ; de l'acide pentaacétique de diéthylènetriamine ; de l'acide tétraphosphonique d'éthylènediaminetétraméthylène, de l'hydrogénophoshate de sodium ; de l'hydrogénophosphate de potassium ; du phosphate de sodium, du phosphate de potassium ; ou des mélanges de ceux-ci.

15. Un procédé de préparation d'un dioxyde de divinylarène comprenant les étapes consistant à :
(A) mettre en contact au moins un divinylarène avec un oxydant de type sel triple de peroxomonosulfate, l'oxydant étant inférieur à 2,0 équivalents par mole de divinylarène ; un solvant ; un composé basique ; et facultativement, avec au moins un catalyseur ; pour produire un produit de dioxyde de divinylarène dans un mélange réactionnel ;
(B) séparer le produit de dioxyde de divinylarène formé à l'étape (A) du mélange réactionnel de l'étape (A) ;
(C) facultativement, purifier le produit de émanationdioxyde de divinylarène obtenu à l'étape (B) ;
(D) facultativement, récupérer et/ou recycler le solvant 5 et le catalyseur du mélange réactionnel de l'étape (A) ; et
(E) facultativement, récupérer et purifier le sel de sous-produit.
